(19) Europäisches Patentamt
European Patent Office
Office européen des brevets

(11) Veröffentlichungsnummer: 0 364 395 A1

# (12) EUROPÄISCHE PATENTANMELDUNG

(21) Anmeldenummer: 89810687.7

(22) Anmeldetag: 14.09.89

(51) Int. Cl.[5]: C07D 271/113 , C07D 285/125 , C07D 413/04 , C07D 417/04 , A01N 43/82 , //(C07D413/04, 333:00,271:00),(C07D413/04, 307:00,271:00),(C07D417/04, 333:00,285:00),(C07D417/04, 307:00,285:00)

Claim for the following Contracting State: ES.

(30) Priorität: 23.09.88 CH 3536/88

(43) Veröffentlichungstag der Anmeldung: 18.04.90 Patentblatt 90/16

(84) Benannte Vertragsstaaten: AT BE CH DE ES FR GB GR IT LI NL

(71) Anmelder: CIBA-GEIGY AG
Klybeckstrasse 141
CH-4002 Basel(CH)

(72) Erfinder: Beriger, Ernst, Dr.
Grabenmattweg 29
CH-4123 Allschwil(CH)
Erfinder: Eckhardt, Wolfgang, Dr.
Breslauerstrasse 14
D-7850 Lörrach(DE)

## (54) Nematizide Mittel.

(57) Es wird ein Verfahren zur Herstellung von 2-Mercapto-1,3,4-oxadiazolen und 2-Mercapto-1,3,4-thiadiazolen der Formel I

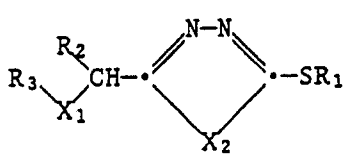

(I)

worin
$X_1$ und $X_2$ voneinander unabhängig Sauerstoff oder Schwefel,
$R_1$ Difluormethyl oder 3,4,4-Trifluor-3-butenyl,
$R_2$ Wasserstoff oder $C_1$-$C_3$-Alkyl,
$R_3$ Wasserstoff, unsubstituiertes oder durch Halogen oder $C_1$-$C_3$-Alkoxy substituiertes $C_1$-$C_5$-Alkyl, unsubstituiertes oder durch Halogen oder $C_1$-$C_3$-Alkoxy substituiertes $C_3$-$C_7$-Alkenyl, unsubstituiertes oder durch Halogen oder $C_1$-$C_3$-Alkoxy substituiertes $C_3$-$C_7$-Alkinyl, unsubstituiertes oder durch Halogen, $NO_2$, $CF_3$, $C_1$-$C_3$-Alkyl oder $C_1$-$C_3$-Alkoxy substituiertes Phenyl bedeuten, worin ferner
$R_2$ und $R_3$ gemeinsam die Methylenkette $(-CH_2-)_n$ als Bestandteil eines 5- oder 6-Rings mit dem Heteroatom $X_1$ bilden können, wobei n 3 oder 4 bedeutet.
Die Verbindungen der Formel I besitzen nematizide Eigenschaften.



Xerox Copy Centre

## Nematizide Mittel

Die vorliegende Erfindung betrifft neue substituierte 2-Mercapto-1,3,4-oxadiazole und 2-Mercapto-1,3,4-thiadiazole, deren Herstellung sowie nematizide Mittel, die als Wirkstoff mindestens eine dieser Verbindungen enthalten. Die Erfindung betrifft die Verwendung von 2-Mercapto-1,3,4-oxadiazole, von 2-Mercapto-1,3,4-thiadiazole und Mittel zur Bekämpfung von Nematoden, insbesondere von pflanzenschädigenden Nematoden.

Die erfindungsgemässen 2-Mercapto-1,3,4-oxadiazole und 2-Mercapto-1,3,4-thiadiazole entsprechen der allgemeinen Formel I

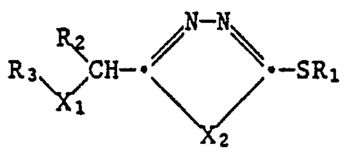

(I)

worin

$X_1$ und $X_2$ voneinander unabhängig Sauerstoff oder Schwefel,

$R_1$ Difluormethyl oder 3,4,4-Trifluor-3-butenyl,

$R_2$ Wasserstoff oder $C_1$-$C_3$-Alkyl,

$R_3$ Wasserstoff, unsubstituiertes oder durch Halogen oder $C_1$-$C_3$-Alkoxy substituiertes $C_1$-$C_5$-Alkyl, unsubstituiertes oder durch Halogen oder $C_1$-$C_3$-Alkoxy substituiertes $C_3$-$C_7$-Alkenyl, unsubstituiertes oder durch Halogen oder $C_1$-$C_3$-Alkoxy substituiertes $C_3$-$C_7$-Alkinyl, unsubstituiertes oder durch Halogen, $NO_2$, $CF_3$, $C_1$-$C_3$-Alkyl oder $C_1$-$C_3$-Alkoxy substituiertes Phenyl bedeuten, worin ferner

$R_2$ und $R_3$ gemeinsam die Methylenkette $(-CH_2-)_n$ als Bestandteil eines 5- oder 6-Rings mit dem Heteroatom $X_1$ bilden können, wobei n 3 oder 4 bedeutet,

sowie Salze einer dieser Verbindungen.

Unter Alkyl sind als selbständiger Rest sowie als Teil einer anderen Gruppe, wie Alkoxy, gerad- und verzweigtkettige Alkylgruppen zu verstehen. Dazu zählen die Methyl-, die Ethyl- sowie die normalen und isomeren Propyl-, Butyl- und Pentylgruppen. Halogensubstituiertes Alkyl steht für einen einfach bis perhalogenierten Alkylrest, wie z.B. $CHCl_2$, $CH_2F$, $CCl_3$, $CH_2Cl$, $CHFCH_3$, $CH_2CH_2Br$, $CF_2CF_3$, $C_2Cl_5$, $CH_2Br$, CHBrCl usw., vorzugsweise für $CHF_2$. Alkenyl bedeutet z.B. Propenyl-(1), Allyl, Butenyl-(1), Butenyl-(2) oder Butenyl-(3) sowie Ketten mit mehreren Doppelbindungen. Halogenalkenyl bedeutet beispielsweise 3,4,4-Trifluor-3-buten-1-yl. Alkinyl bedeutet z.B. Propinyl-(2), Butinyl-(1), Butinyl-(2), Pentinyl-(4) usw.. Halogen steht für Fluor, Chlor, Brom oder Jod, vorzugsweise für Fluor, Chlor oder Brom.

Beispiele salzbildender Säuren sind unter den anorganischen Säuren Halogenwasserstoffsäuren wie Fluorwasserstoffsäure, Chlorwasserstoffsäure, Bromwasserstoffsäure oder Jodwasserstoffsäure sowie ferner Schwefelsäure, Phosphorsäure, phosphorige Säure, Salpetersäure und unter den organischen Säuren Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Es sind bereits Oxadiazol- und Thiadiazol-Derivate bekannt, die als nematizid wirksam beschrieben sind. So sind in der US-Patentschrift 3,770,754 solche Verbindungen mit einer 1,2,4-Stellung der Heteroatome offenbart, während in der US-Patentschrift 4,454,147 1,3,4-Thiadiazol-Derivate beschrieben sind, bei denen im Vergleich mit den erfindungsgemässen Verbindungen der Heterocyclus anstelle der Mercapto-Gruppen durch ein Chloratom substituiert ist. Diese bekannten Verbindungen haben bisher als Nematizide die in der Praxis an sie gestellten Ansprüche nicht in vollem Umfang befriedigen können. Ferner sind Oxadiazol-Derivate mit fungizider Wirksamkeit in der DE-OS 2 361 613 beschrieben. Es werden darin jedoch keine dieser Verbindungen expressis verbis genannt, die unter den Umfang der erfindungsgemässen Formel I fallen.

Durch die Bereitstellung der erfindungsgemässen Verbindungen der Formel I ist es nun gelungen einen wertvollen Beitrag zur Bekämpfung der beträchtliche landwirtschaftliche Schäden an Pflanzgut verursachenden Pflanzennematoden zu leisten. Auf diese Weise können Ernteeinbussen bei Kulturpflanzen wie z.B. Kartoffeln, Getreide, Rüben, Raps, Kohl, Tabak, Sojabohnen, Baumwolle und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung von wurzelparasitären Bodennematoden wie z.B. solchen der Gattungen Heterodera und Globodera (zystenbildende Nematoden), Meloidogyne (Wurzelgallennematoden) sowie der Gattungen Radopholus, Pratylenchus, Tylenchulus, Longidorus, Trichodorus und Xiphinema aus. Ferner lassen sich mit den erfindungsgemässen Wirkstoffen die Nematodengattungen. Ditylenchus (Stengelparasiten), Aphelenchoides (Blattnematoden) und Anguina (Blütennematoden) wirkungsvoll bekämpfen.

Mit den Wirkstoffen der Formel lassen sich vorzugsweise besonders schädliche Nematodenarten der Gattung Meloidogyne, wie z.B. Meloidogyne incognita, sowie der Gattung Heterodera, wie z.B. Heterodera glycines (Sojabohnen-Zystennematode) und ferner der Gattung Globodera, wie z.B. Globodera rostochiensis (Kartoffelzystennematode) sowie Vertreter von wandernden Endoparasiten, wie z.B. Pratylenchus penetrans oder Radopholus similis und Vertreter von Ektoparasiten, wie z.B. Trichodorus spp. und Xiphinema spp. erfolgreich bekämpfen.

Zur Bekämpfung der Pflanzennematoden und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematodenspecies und werden somit den Erfordernissen der Praxis gerecht. Die nematizide Wirkungsweise der erfindungsgemässen Verbindungen wird durch eine geringe Phytotoxizität in vorteilhafter Weise begleitet, womit der allgemein wünschenswerten Verminderung der Umweltbelastung in besonderem Masse Rechnung getragen wird.

Im Rahmen der vorliegenden Erfindung sind jene 2-Mercapto-1,3,4-oxadiazole der Formel Ia

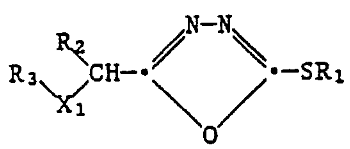

(Ia),

bevorzugt, bei welchen
$R_1$ Difluormethyl,
$R_2$ Wasserstoff und
$R_3$ Methyl bedeuten, oder worin
$R_2$ und $R_3$ zusammen die Methylenkette $(-CH_2-)_3$, als Bestandteil eines Tetrahydrofuran-2-yl- oder eines Tetrahydrothienylrings bilden.

Das 2-Difluormethylthio-5-(tetrahydrofuran-2-yl)-1,3,4-oxadiazol ist als Einzelverbindung dabei hervorzuheben.

Bevorzugt sind auch jene 2-Mercapto-oxadiazole der Formel Ia, bei welchen
$R_1$ 3,4,4-Trifluor-3-butenyl,
$R_2$ Wasserstoff und
$R_3$ Methyl bedeuten, oder worin
$R_2$ und $R_3$ zusammen die Methylenkette $(-CH_2-)_3$, als Bestandteil eines Tetrahydrofuran-2-yl- oder eines Tetrahydrothienylrings bilden.

Das 2-(3,4,4-Trifluormethyl-3-butenylthio)-5-(tetrahydrofuran-2-yl)-1,3,4-oxadiazol ist als Einzelverbindung dabei hervorzuheben.

Es werden ferner jene 2-Mercapto-1,3,4-thiadiazole der Formel Ib

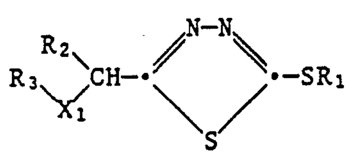

(Ib),

bevorzugt, worin $R_1$ Difluormethyl, $R_2$ Wasserstoff, $R_3$ unsubstituiertes oder durch Halogen substituiertes $C_1$-$C_3$-Alkyl oder unsubstituiertes oder durch Halogen substituiertes Phenyl bedeutet.

Als Einzelverbindungen sind dabei das 2-Difluormethylthio-5-methoxymethyl-1,3,4-thiadiazol und das 2-Difluormethylthio-5-(4-chlorphenoxymethyl)-1,3,4-thiadiazol hervorzuheben.

Die Verbindungen der Formel werden erfindungsgemäss hergestellt, indem man a) in einer Kondensationsreaktion eine Verbindung der Formel II

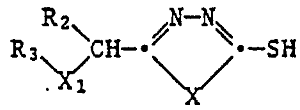

(II)

oder eine Verbindung der Formel III

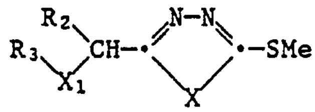

(III)

mit einer Verbindung der Formel IV

Hal-$R_1$ (IV)

in einem inerten Lösungsmittel oder Lösungsmittel-Gemisch bei Raumtemperatur oder bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

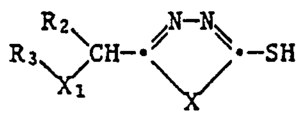

(II)

mit einer Verbindung der Formel V

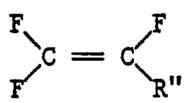

(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ic

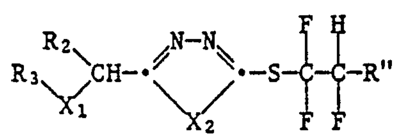

(Ic)

oder zu einer Verbindung der Formel Id

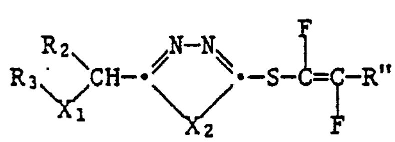

(Id)

führt, wobei in den vorstehend genannten Formeln II, III, Ic, Id, IV und V Me für ein Alkalimetall oder für Ammonium steht, Hal Halogen, vorzugsweise Chlor, Brom oder Jod bedeutet, und R″ Fluor oder Trifluormethyl darstellt, während $R_1$, $R_2$, $R_3$, $X_1$ und $X_2$ die unter Formel I angegebenen Bedeutungen besitzen.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid, Ketone wie Aceton, Diethylketon, Methylethylketon

sowie Wasser und Alkohole wie z.B. Methanol, Ethanol, Isopropanol oder Butanol; und ganz allgemein Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriumethylat.

Die Zugabe katalytischer Mengen eines Kronenethers, wie z.B. 18-Krone-6 oder 15-Krone-5, wirkt sich in den Herstellungsverfahren günstig auf den Reaktionsablauf aus. Ferner hat sich für den gleichen Zweck die katalytische Verwendung von Tetraalkylaminsalzen, z.B. Tetraalkylammoniumchloride oder -bromide, vorzugsweise Tetra-n-butylammoniumbromid, als vorteilhaft erwiesen. Darüber hinaus sind Alkalijodide, vorzugsweise Kaliumjodid, als Katalysatoren vorteilhaft einsetzbar.

In den Herstellungsverfahren betragen die Reaktionstemperaturen 10 bis 90°C, vorzugsweise 300 bis 80°C. Für die Druckverhältnisse während des Reaktionsablaufs sind 1 bis 20 bar, vorzugsweise 6 bis 14 bar, massgebend.

Die Ausgangsverbindungen der Formel II sind teils bekannt, teils neu. Die neuen Verbindungen der genannten Formeln sind Zwischenprodukte zur Herstellung wertvoller nematozider Wirkstoffe (siehe Tabellen 0-3) und bilden damit einen Bestandteil der vorliegenden Erfindung.

Die Ausgangsverbindungen der Formel Ia und Ib können nach bekannten Methoden wie folgt hergestellt werden:

a) Die 2-Mercapto-1,3,4-oxadiazole sind gewinnbar durch Zugabe von Schwefelkohlenstoff zu einer Lösung des entsprechend substituierten Hydrazids in alkoholisch-wässrigem Kaliumhydroxid und Erhitzen des Reaktionsgemisches während einiger Stunden. Als Lösungsmittel werden dabei Alkohole wie z.B. Ethylalkohol oder n-Amylalkohol verwendet. Durch Ansäuern der gebildeten Kaliumsalze werden die freien Mercapto-Verbindungen erhalten [vgl. J.Am.Chem.Soc. 78, 4975-4978 (1956)]. Die 2-Mercapto-1,3,4-oxadiazole sind ferner gewinnbar durch Umsetzung des entsprechenden Acylhydrazids mit Thiophosgen in einem inerten Lösungsmittel wie z.B. Dioxan [vgl. J.Org.Chem. 26, 88-95 (1961)].

b) Die 2-Mercapto-1,3,4-thiadiazole sind erhältlich durch Behandlung des entsprechend substituierten Acyl-kaliumdithiocarbazats mit konzentrierter Schwefelsäure bei -5° bis 10°C [vgl. J.prakt.Chem. 93, 49 (1916); J.Org.Chem. 23, 1021 (1958); J.Heterocycl.Chem. 19, 542-544 (1982)].

Die Erfindung betrifft auch Mittel, zur Bekämpfung von pflanzenschädigenden Nematoden sowie zur präventiven Verhütung des Nematodenbefalls von Pflanzgut, welche die Wirkstoffe der Formel I enthalten.

Darüber hinaus schliesst die vorliegende Erfindung zusätzlich die Herstellung nematizider Mittel ein, die gekennzeichnet ist durch das innige Vermischen von Aktivsubstanzen der Formel I mit einer oder mehreren hierin beschriebenen Trägerstoffen und Hilfsingredienzen. Eingeschlossen ist auch ein Verfahren zur Behandlung von Pflanzen, das durch die Applikation der Verbindungen der Formel I oder der neuen Mittel charakterisiert ist.

Ein bezvorzugtes Verfahren für den Einsatz eines Wirkstoffs der Formel I bzw. eines nematiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel I können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel, Knospen oder Blätter.

Wirkstoffe der Formel I werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a., oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Die Verbindungen der Formel I werden in unveränderter Form oder vorzugsweise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern,

Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 500 g bis 6 kg Aktivsubstanz (AS) je ha; bevorzugt 1 bis 4 kg AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien genannt die Alkali-, Erdalkali- oder gegebenenfalls substituierten Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise zwei Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat

in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:
"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien.

Die agrochemischen Zubereitungen enthalten in der Regel 0,1 bis 99 Gew.-%, insbesondere 0,1 bis 95 Gew.-% Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

## 1. Herstellungsbeispiele

### H.1 2-Difluormethylthio-5-(4-chlorphenoxymethyl)-1,3,4-thiadiazol (Verbindung Nr. 1.3)

2 g Kaliumhydroxid werden in 8 ml Wasser gelöst und mit 30 ml Dioxan verdünnt. Dazu gibt man unter Rühren 5,2 g 2-Mercapto-5-(4-chlorphenoxymethyl)-1,3,4-thiadiazol. Nach Zugabe von 0,1 g Kaliumjodid und 0,1 g Tetrabutylammoniumbromid leitet man bei 40 °C Badtemperatur, immer unter starkem Rühren, Chlordifluormethan ein, bis die Aufnahme beendet ist. Die Reaktionsmischung wird im Vakuum eingedampft, der Rückstand in Methylenchlorid aufgenommen und nacheinander mit Wasser und mit 1-n Natronlauge ausgewaschen. Nach dem Abdampfen des Lösungsmittels erhält man die Titelverbindung in kristalliner Form vom Schmelzpunkt 56-57 °C (aus Aether).

### H.2 2-(3,4,4-Trifluor-3-buten-1-ylthio)-5-(1′-thiomethyl-methyl)-1,3,4-oxadiazol (Verbindung Nr. 2.1)

3,3 g 2-Mercapto-5-methoxymethyl-1,3,4-oxadiazol werden zusammen mit 2,3 g Kalium-t-butylat in einer Mischung von 25 ml Dioxan und 5 ml Dimethylformamid verrührt. Man lässt bei Raumtemperatur 4,5 g 4-Brom-1,1,2-trifluor-buten-1 zutropfen und rührt noch einige Stunden bei Raumtemperatur nach. Nach üblicher Aufarbeitung erhält man 3,8 g der Titelverbindung in Form eines Oeles, $n_D^{27}$ = 1,5091.

### H.3 2-(3,4,4-Trifluor-3-buten-1-ylthio)-5-(tetrahydrofuran-2-yl)-1,3,4-oxadiazol (Verbindung Nr. 3.6)

In einer Lösung von 3,2 g (0,018 Mol) 2-Mercapto-5-(tetrahydrofuran-2-yl)-1,3,4-oxadiazol in einem Gemisch von 80 ml 1,4-Dioxan und 10 ml Dimethylformamid werden 2,1 g (0,019 Mol) Kalium-t-butylat gegeben. Anschliessend werden 4,9 g (0,026 Mol) 1-Brom-3,4,4-trifluor-3-buten bei 25 °C zugetropft und das Reaktionsgemisch 16 Stunden bei Zimmertemperatur gerührt. Das Lösungsmittelgemisch wird unter vermindertem Druck abdestilliert und der Rückstand in Essigsäureethylester gelöst. Diese Lösung wird nacheinander mit Wasser, eiskalter 1N Natronlauge und Wasser extrahiert, mit Natriumsulfat getrocknet, filtriert und das Lösungsmittel unter vermindertem Druck abdestilliert. Man erhält 80,43 g des gewünschten Produktes als gelbes Oel ($n_D^{23}$ = 1,4904).

Analog der vorstehenden Herstellungsbeispiele sowie der vorbeschriebenen Verfahren lassen sich folgende erfindungsgemässe Verbindungen herstellen (Tabellen O-3). Die nachfolgend aufgeführten Verbindungen dienen der Illustration der vorliegenden Erfindung und stellen keine Begrenzung derselben dar.

Tabelle 0

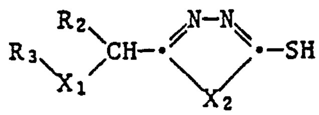

| Verb. Nr. | $R_3$ | $R_2$ | $X_1$ | $X_2$ | Physik. Daten |
|---|---|---|---|---|---|
| 0.1 | $CH_3$ | H | O | S | Smp. 77- 79°C |
| 0.2 | H | H | O | S | Smp. 115-120°C |
| 0.3 | 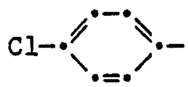 | H | O | S | Smp. 155-158°C |
| 0.4 | $CH_3$ | H | S | O | Smp. 67- 69°C |

Tabelle 1

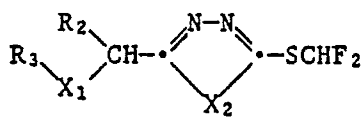

| Verb. Nr. | $R_3$ | $R_2$ | $X_1$ | $X_2$ | Physik. Daten |
|---|---|---|---|---|---|
| 1.1 | $CH_3$ | H | O | S | $n_D^{22}$ = 1,5182 |
| 1.2 | H | H | O | S | $n_D^{22}$ = 1,5308 |
| 1.3 | 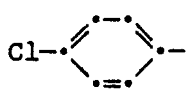 | H | O | S | Smp. 56- 57°C |
| 1.4 | $CH_3$ | H | S | O | $n_D^{25}$ = 1,5219 |

Tabelle 2

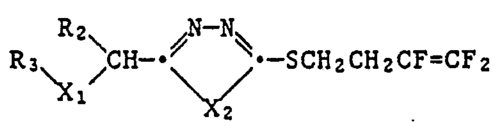

| Verb. Nr. | $R_3$ | $R_2$ | $X_1$ | $X_2$ | Physik. Daten |
|---|---|---|---|---|---|
| 2.1 | $CH_3$ | H | S | O | $n_D^{27}$ = 1,5091 |
| 2.2 | H | H | O | S | |
| 2.3 | $CH_3$ | H | O | S | $n_D^{22}$ = 1,5125 |
| 2.4 | 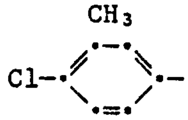 | H | O | S | $n_D^{22}$ = 1,5498 |

Tabelle 3

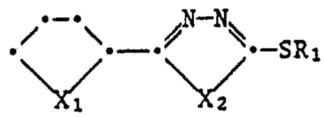

| Verb. Nr. | $R_1$ | $X_1$ | $X_2$ | Physik. Daten |
|---|---|---|---|---|
| 3.1. | $-CHF_2$ | O | S | $n_D^{23}$ = 1,5373 |
| 3.2. | $-CHF_2$ | O | O | $n_D^{23}$ = 1,4940 |
| 3.3. | $-CHF_2$ | S | O | |
| 3.4. | $-CHF_2$ | S | S | |
| 3.5. | $-CH_2CH_2CF=CF_2$ | O | S | |
| 3.6. | $-CH_2CH_2CF=CF_2$ | O | O | $n_D^{23}$ = 1,4904 |
| 3.7. | $-CH_2CH_2CF=CF_2$ | S | O | Oel |
| 3.8. | $-CH_2CH_2CF=CF_2$ | S | S | $n_D^{50}$ = 1,5831 |

## F. Formulierungsbeispiele

### F.2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen 0-3 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Korzentration hergestellt werden.

| 2.2 Lösungen | a) | b) | c) | d) |
|---|---|---|---|---|
| Wirkstoff aus den Tabellen 0-3 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff aus den Tabellen 0-3 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 0-3 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

F.3 Formulierungsbeispiele für feste Wirkstoffe der Formel I (% = Gewichtsprozent)

| 3.1 Spritzpulver | a) | b) | c) |
|---|---|---|---|
| Wirkstoff aus den Tabellen 0-3 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 3.2 Emulsions-Konzentrat | |
|---|---|
| Wirkstoff aus den Tabellen 0-3 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3.3 Stäubemittel | a) | b) |
|---|---|---|
| Wirkstoff aus den Tabellen 0-3 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| 3.4 Extruder Granulat | |
|---|---|
| Wirkstoff aus den Tabellen 0-3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 3.5 Umhüllungs-Granulat | |
|---|---|
| Wirkstoff aus den Tabellen 0-3 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 3.6 Suspensions-Konzentrat | |
|---|---|
| Wirkstoff aus den Tabellen 0-3 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

B. Biologisches Beispiel

B.3.1 Wirkung gegen Meloidogyne incognita auf Tomaten

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte

Tomatenpflanze gepflanzt und der formulierte Wirkstoff mittels Drenchapplikation in die Töpfe hineingegeben (0,0006 % Aktivsubstanz bezogen auf das Bodenvolumen). Die eingetopften Pflanzen werden nun bei einer Temperatur von 26±1 °C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgallen-Index ("Root-Knot Index").

Verbindungen aus Tabelle 1 zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmen z.B. die Verbindungen Nr. 1.1, 1.3, 3.2, 3.6, 3.7 und 3.8 im obigen Versuch die Wurzelgallbildung fast vollständig (0-10 % Restbefall).

## Ansprüche

1. 2-Mercapto-1,3,4-oxadiazole und 2-Mercapto-1,3,4-thiadiazole der Formel I

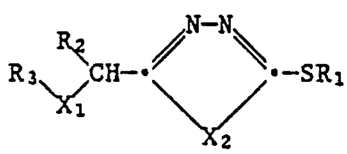

(I),

worin
$X_1$ und $X_2$ voneinander unabhängig Sauerstoff oder Schwefel,
$R_1$ Difluormethyl oder 3,4,4-Trifluor-3-buten-1-yl,
$R_2$ Wasserstoff oder $C_1$-$C_3$-Alkyl,
$R_3$ Wasserstoff, unsubstituiertes oder durch Halogen oder $C_1$-$C_3$-Alkoxy substituiertes $C_1$-$C_5$-Alkyl, unsubstituiertes oder durch Halogen oder $C_1$-$C_3$-Alkoxy substituiertes $C_3$-$C_7$-Alkenyl, unsubstituiertes oder durch Halogen oder $C_1$-$C_3$-Alkoxy substituiertes $C_3$-$C_7$-Alkinyl, unsubstituiertes oder durch Halogen, $NO_2$, $CF_3$, $C_1$-$C_3$-Alkyl oder $C_1$-$C_3$-Alkoxy substituiertes Phenyl bedeuten, worin ferner
$R_2$ und $R_3$ gemeinsam die Methylenkette $(-CH_2-)_n$ als Bestandteil eines 5- oder 6-Rings mit dem Heteroatom $X_1$ bilden können, wobei n 3 oder 4 bedeutet.

2. 2-Mercapto-1,3,4-oxadiazole der Formel Ia

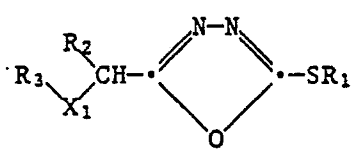

(Ia),

gemäss Anspruch 1, worin
$R_1$ Difluormethyl,
$R_2$ Wasserstoff und
$R_3$ Methyl bedeuten, oder worin
$R_2$ und $R_3$ zusammen die Methylenkette $(-CH_2-)_3$, als Bestandteil eines Tetrahydrofuran-2-yl- oder eines Tetrahydrothienylrings bilden.

3. Das 2-Difluormethylthio-5-(tetrahydrofuran-2-yl)-1,3,4-oxadiazol gemäss Anspruch 2.

4. 2-Mercapto-1,3,4-oxadiazole der Formel Ia

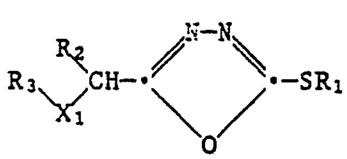

(Ia),

gemäss Anspruch 1, worin
$R_1$ 3,4,4-Trifluor-3-buten-1-yl

$R_2$ Wasserstoff und
$R_3$ Methyl bedeuten, oder worin
$R_2$ und $R_3$ zusammen die Methylenkette $(-CH_2-)_3$, als Bestandteil eines Tetrahydrofuran-2-yl- oder eines Tetrahydrothienylrings bilden.

5. Das 2-(3,4,4-Trifluor-3-buten-1-ylthio)-5-(tetrahydrofuran-2-yl)-1,3,4-oxadiazol gemäss Anspruch 4.

6. 2-Mercapto-1,3,4-thiadiazole der Formel Ib

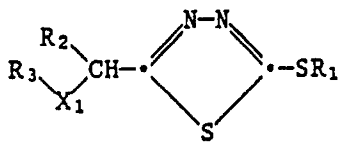
(Ib),

gemäss Anspruch 1, worin
$R_1$ Difluormethyl,
$R_2$ Wasserstoff,
$R_3$ unsubstituiertes oder durch Halogen substituiertes $C_1$-$C_3$-Alkyl oder unsubstituiertes oder durch Halogen substituiertes Phenyl bedeutet.

7. Das 2-Difluormethylthio-5-methoxymethyl-1,3,4-thiadiazol gemäss Anspruch 6.

8. Das 2-Difluormethylthio-5-(4-chlorphenoxy-methyl)-1,3,4-thiadiazol gemäss Anspruch 6.

9. Verfahren zur Herstellung der Verbindungen der Formel I gemäss Anspruch 1, dadurch gekennzeichnet, dass man

a) in einer Kondensationsreaktion eine Verbindung der Formel II

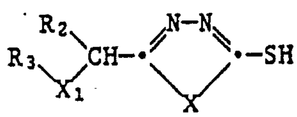
(II)

oder eine Verbindung der Formel III

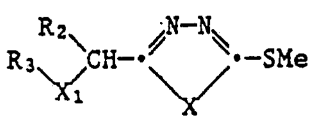
(III)

mit einer Verbindung der Formel IV
Hal-$R_1$ (IV)
in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

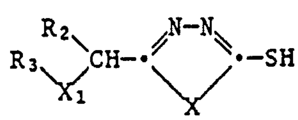
(II)

mit einer Verbindung der Formel V

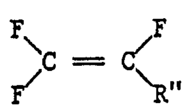
(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese

Reaktion zu einer Verbindung der Formel Ic

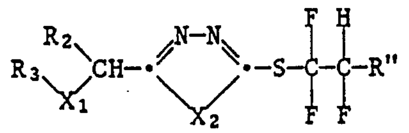

(Ic)

oder zu einer Verbindung der Formel Id

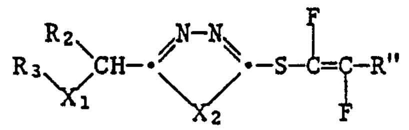

(Id)

führt, wobei in den vorstehend genannten Formeln II, III, Ic, Id, IV und V Me Für ein Alkalimetall oder für Ammonium steht, Hal Halogen, vorzugsweise Chlor, Brom oder Jod bedeutet, und R″ Fluor oder Trifluormethyl darstellt, während $R_1$, $R_2$, $R_3$, $X_1$ und $X_2$ die unter Formel I angegebenen Bedeutungen besitzen.

10. Schädlingsbekämpfungsmittel zur Bekämpfung oder Verhütung des Befalls von Pflanzen durch Nematoden, dadurch gekennzeichnet, dass es als aktive Komponente mindestens eine Verbindung der Formel I, gemäss Anspruch 1 enthält.

11. Mittel gemäss Anspruch 10, dadurch gekennzeichnet, dass es als aktive Komponente mindestens eine Verbindung der Formel I gemäss der Ansprüche 2 bis 8 enthält.

12. Mittel gemäss Anspruch 10, dadurch gekennzeichnet, dass es als aktive Komponente 2-Difluormethylthio-5-methoxymethyl-1,3,4-thiadiazol enthält.

13. Mittel gemäss Anspruch 10, dadurch gekennzeichnet, dass es als aktive Komponente 2-(3,4,4-Trifluor-3-buten-1-ylthio)-5-(tetrahydrofuran-2-yl)-1,3,4-oxadiazol enthält.

14. Mittel nach einem der Ansprüche 10 bis 13, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

15. Mittel nach Anspruch 14, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

16. Verfahren zur Herstellung eines agrochemischen Mittels, dadurch gekennzeichnet, dass mindestens eine Verbindung der Formel I gemäss Anspruch 1 mit geeigneten festen oder flüssigen Zusatzstoffen und Tensiden innig vermischt.

17. Verfahren zur Bekämpfung oder Verhütung eines Befalls von Kulturpflanzen durch Nematoden, dadurch gekennzeichnet, dass man eine Verbindung der Formel I gemäss Anspruch 1 auf die Pflanze oder deren Standort appliziert.

18. Verwendung gemäss Anspruch 17, von Verbindungen der Formel I gemäss einem der Ansprüche 2 bis 8.

19. Verwendung gemäss Anspruch 18, dadurch gekennzeichnet, dass es sich bei den Nematoden um pflanzenparasitäre Arten handelt.

20. Verwendung gemäss Anspruch 19 gegen Nematoden der Gattung Meloidogyne, Heterodera oder Globodera.

Patentansprüche für folgenden Vertragsstaat: ES

1. Verfahren zur Herstellung von 2-Mercapto-1,3,4-oxadiazolen und 2-Mercapto-1,3,4-thiadiazolen der Formel I

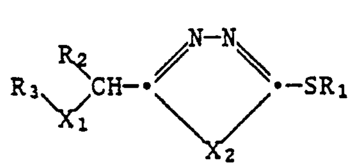

(I),

worin

$X_1$ und $X_2$ voneinander unabhängig Sauerstoff oder Schwefel,
$R_1$ Difluormethyl oder 3,4,4-Trifluor-3-buten-1-yl,
$R_2$ Wasserstoff oder $C_1$-$C_3$-Alkyl,
$R_3$ Wasserstoff, unsubstituiertes oder durch Halogen oder $C_1$-$C_3$-Alkoxy substituiertes $C_1$-$C_5$-Alkyl, unsubstituiertes oder durch Halogen oder $C_1$-$C_3$-Alkoxy substituiertes $C_3$-$C_7$-Alkenyl, unsubstituiertes oder durch Halogen oder $C_1$-$C_3$-Alkoxy substituiertes $C_3$-$C_7$-Alkinyl, unsubstituiertes oder durch Halogen, $NO_2$, $CF_3$, $C_1$-$C_3$-Alkyl oder $C_1$-$C_3$-Alkoxy substituiertes Phenyl bedeuten, worin ferner
$R_2$ und $R_3$ gemeinsam die Methylenkette $(-CH_2-)_n$ als Bestandteil eines 5- oder 6-Rings mit dem Heteroatom $X_1$ bilden können, wobei n 3 oder 4 bedeutet, dadurch gekennzeichnet, dass man

a) in einer Kondensationsreaktion eine Verbindung der Formel II

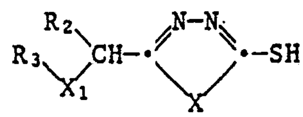
(II)

oder eine Verbindung der Formel III

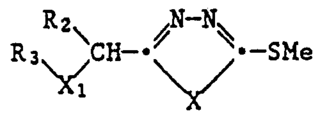
(III)

mit einer Verbindung der Formel IV
Hal-$R_1$ (IV)
in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel II in Gegenwart einer Base verläuft, oder

b) in einer Anlagerungsreaktion eine Verbindung der Formel II

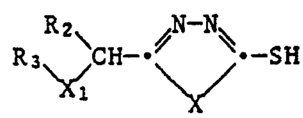
(II)

mit einer Verbindung der Formel V

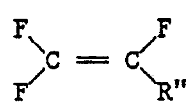
(V)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ic

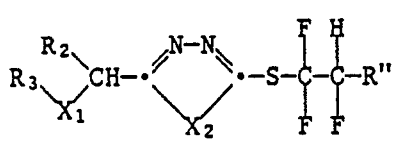
(Ic)

oder zu einer Verbindung der Formel Id

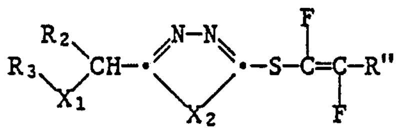 (Id)

führt, wobei in den vorstehend genannten Formeln II, III, Ic, Id, IV und V Me für ein Alkalimetall oder für Ammonium steht, Hal Halogen, vorzugsweise Chlor, Brom oder Jod bedeutet, und R″ Fluor oder Trifluormethyl darstellt.

2. Verfahren zur Herstellung von 2-Mercapto-1,3,4-oxadiazolen der Formel Ia

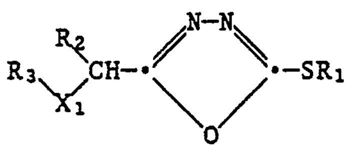 (Ia),

gemäss Anspruch 1, worin
$R_1$ Difluormethyl,
$R_2$ Wasserstoff und
$R_3$ Methyl bedeuten, oder worin
$R_2$ und $R_3$ zusammen die Methylenkette $(-CH_2-)_3$, als Bestandteil eines Tetrahydrofuran-2-yl- oder eines Tetrahydrothienylrings bilden.

3. Verfahren zur Herstellung von 2-Difluormethylthio-5-(tetrahydrofuran-2-yl)-1,3,4-oxadiazol gemäss Anspruch 2.

4. Verfahren zur Herstellung von 2-Mercapto-1,3,4-oxadiazolen der Formel Ia

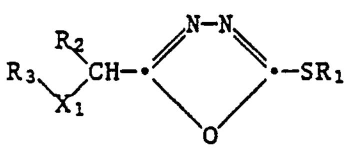 (Ia),

gemäss Anspruch 1, worin
$R_1$ 3,4,4-Trifluor-3-buten-1-yl
$R_2$ Wasserstoff und
$R_3$ Methyl bedeuten, oder worin
$R_2$ und $R_3$ zusammen die Methylenkette $(-CH_2-)_3$, als Bestandteil eines Tetrahydrofuran-2-yl- oder eines Tetrahydrothienylrings bilden.

5. Verfahren zur Herstellung von 2-(3,4,4-Trifluor-3-buten-1-ylthio)-5-(tetrahydrofuran-2-yl)-1,3,4-oxadiazol gemäss Anspruch 4.

6. Verfahren zur Herstellung von 2-Mercapto-1,3,4-thiadiazolen der Formel Ib

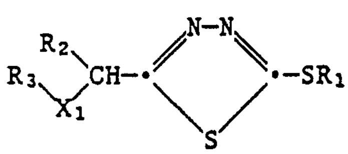 (Ib),

gemäss Anspruch 1, worin
$R_1$ Difluormethyl,
$R_2$ Wasserstoff,
$R_3$ unsubstituiertes oder durch Halogen substituiertes $C_1$-$C_3$-Alkyl oder unsubstituiertes oder durch Halogen substituiertes Phenyl bedeutet.

7. Verfahren zur Herstellung von 2-Difluormethylthio-5-methoxymethyl-1,3,4-thiadiazol gemäss Anspruch 6.

8. Verfahren zur Herstellung von 2-Difluormethylthio-5-(4-chlorphenoxymethyl)-1,3,4-thiadiazol gemäss Anspruch 6.

9. Verfahren zur Bekämpfung oder Verhütung eines Befalls von Kulturpflanzen durch Nematoden, dadurch gekennzeichnet, dass man eine Verbindung der Formel I gemäss Anspruch 1 auf die Pflanze oder deren Standort appliziert.

10. Verwendung gemäss Anspruch 9, dadurch gekennzeichnet, dass es sich bei den Nematoden um pflanzenparasitäre Arten handelt.

11. Verwendung gemäss Anspruch 10 gegen Nematoden der Gattung Meloidogyne, Heterodera oder Globodera.

Europäisches Patentamt

# EUROPÄISCHER RECHERCHENBERICHT

Nummer der Anmeldung

EP 89 81 0687

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | WO-A-8 607 590 (FMC CORP.)<br>* Seite 81, Anspruch 1; Seite 82, Anspruch 5; Seite 85, Anspruch 18 * | 1,10 | C 07 D 271/113<br>C 07 D 285/125<br>C 07 D 413/04<br>C 07 D 417/04<br>A 01 N 43/82 //<br>(C 07 D 413/04<br>C 07 D 333:00<br>C 07 D 271:00 )<br>(C 07 D 413/04<br>C 07 D 307:00<br>C 07 D 271:00 )<br>(C 07 D 417/04<br>C 07 D 333:00<br>C 07 D 285:00 )<br>(C 07 D 417/04<br>C 07 D 307:00<br>C 07 D 285:00 ) |
| A | EP-A-0 263 066 (CIBA-GEIGY)<br>* Seite 18, Anspruch 1; Seite 20, Anspruch 11 * | 1,10 | |
| A | EP-A-0 239 047 (MITSUBISHI)<br>* Seite 20, Anspruch 1; Seite 21, Anspruch 8 * | 1,10 | |
| A | US-A-4 259 104 (L.H. EDWARDS)<br>* Spalte 8, Ansprüche 1,2 * | 1,10 | |
| A | THE JOURNAL OF ORGANIC CHEMISTRY Band 26, Nr. 1, Januar 1961, Seiten 88-95, Easton, PA, USA; W.R. SHERMAN: "5-Nitro-furyl-substituted 1,3,4-Oxadiazoles, 1,3,4-Thiadiazoles, and 1,3,5-Triazines" * Seite 92, Spalte 2, Verbindung 9 * | 1,10 | RECHERCHIERTE SACHGEBIETE (Int. Cl.5) |
| A | ANNALI DI CHIMICA Band 53, Nr. 11, 1963, Seiten 1687-1696; O. TURILLI et al.: "1,3,4-ossadiazoli-2-tiol-5-eterociclo-sostituiti" * Seiten 1693,1694, Verbindungen IIIa,IIIb * | 1 | C 07 D 271/00<br>C 07 D 285/00<br>C 07 D 413/00<br>C 07 D 417/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 18-12-1989 | KYRIAKAKOU G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument